# EUROPEAN PATENT APPLICATION

(11) **EP 4 454 631 A1**
(43) Date of publication of application: **30.10.2024**
(21) Application number: 22911313.9
(22) Date of filing: 21.12.2022
(51) Int. Cl.: A61K 6/887, A61K 6/836

(54) **CURABLE DENTAL COMPOSITION**

(30) Priority: 21.12.2021 JP 2021207553
(71) Applicant: Kuraray Noritake Dental Inc., Kurashiki-shi, Okayama 710-0801 (JP)
(72) Inventor: SUZUKI, Kenji, Tainai-shi, Niigata 959-2653 (JP); ISHIBASHI, Misaki, Tainai-shi, Niigata 959-2653 (JP)
(74) Representative: D Young & Co LLP
(86) International application number: PCT/JP2022/047233
(87) International publication number: WO 2023/120611

(57) **Abstract**

The present invention relates to a dental curable composition containing a (meth)acrylic based monomer (A) containing two or more (meth)acryloyloxy groups and one or more urethane bond in one molecule, a (meth)acrylate ester compound (B) containing two or more (meth)acryloyloxy groups and one or more hydroxy group in one molecule, a polymerization initiator (C), and an ion releasing compound (D); a dental filling and restorative material containing the dental curable composition; a dental cement containing the dental curable composition; and a dental abutment building material containing the dental curable composition.

## Description

### Technical Field

The present invention relates to a dental curable composition that is suitable as a dental filling and restorative material, a dental cement, and a dental abutment building material.

### Background Art

A dental composite resin constituted mainly by a polymerizable monomer, a filler, and a polymerization initiator has become the most frequently used dental material for restoring a defective part of a tooth or dental caries, or for bonding dental prostheses. Examples of the dental material include a dental filling and restorative material, a dental cement, and a dental abutment building material.

Secondary caries occurs by bacteria invading into a construction gap caused due to improper treatment, delamination of the dental composite resin from the adhesion surface, or deterioration of the dental composite resin on the adhesion surface, and there is a strong demand of preventing the secondary caries as much as possible.

Examples of the method of preventing the secondary caries generally include a method of reinforcing the tooth substance by forming an acid resistant layer. Examples of the method of reinforcing the tooth substance include supplying an ion, such as a fluoride ion, to the tooth substance, and specifically an ion releasing compound is mixed in the dental composite resin, or a material obtained by mixing an inorganic compound that is referred to as a glass ionomer and a polycarboxylic acid is used.

However, in the case where the ion releasing compound is mixed in the dental composite resin, there is a problem that not only the sufficient ion releasability cannot be obtained, but also the strength and the adhesion durability are deteriorated due to such factors as water absorption and the like. Furthermore, the glass ionomer is excellent in ion releasability, but has a problem of low strength and low adhesiveness.

As a technique for achieving the strength while preventing the secondary caries, for example, NPL 1 describes a dental composite resin using a dimethacrylate containing a urethane bond, 2-hydroxyethyl methacrylate, a polycarboxylic acid, and fluoroaluminosilicate glass.

### Citation List

### Non-patent Literature

NPL 1: Osvaldo Zmener, et al., Resistance against bacterial leakage of four luting agents used for cementation of complete cast crowns, American Journal of Dentistry, February, 2014, Vol. 27, No. 1, pp. 51-55

### Summary of Invention

### Technical Problem

However, as a result of the investigations by the present inventors, it has been found that the dental curable composition described in NPL 1 can achieve certain ion releasability, but the mechanical strength thereof is in a low level. It has also been found that the polymerizable monomer has a high polarity due to the achievement of the ion releasability, resulting in significantly low adhesiveness particularly after storing in water for a long period of time, and there is room for improvement in the mechanical strength and the adhesion durability.

Under the circumstances, an object of the present invention is to provide a dental curable composition that has an ion releasability, and is excellent in mechanical strength and adhesion durability.

### Solution to Problem

The present invention encompasses the following inventions.
[1] A dental curable composition containing a (meth)acrylic based monomer (A) containing two or more (meth)acryloyloxy groups and one or more urethane bond in one molecule, a (meth)acrylate ester compound (B) containing two or more (meth)acryloyloxy groups and one or more hydroxy group in one molecule, a polymerization initiator (C), and an ion releasing compound (D).
[2] The dental curable composition according to the item [1], in which the dental curable composition further contains a mono(meth)acrylate ester compound (E) that does not contain any of a hydroxy group, an acidic group, an amino group, and an amide group in one molecule.
[3] The dental curable composition according to the item [1] or [2], in which the dental curable composition further contains a polymerization accelerator (F).
[4] The dental curable composition according to any one of the items [1] to [3], in which the dental curable composition further contains a filler (G).
[5] The dental curable composition according to any one of the items [1] to [4], in which the dental curable composition contains substantially no polycarboxylic acid.
[6] The dental curable composition according to any one of the items [1] to [5], in which the component (D) contains one kind selected from the group consisting of a fluoride ion releasing compound and a calcium ion releasing compound.
[7] The dental curable composition according to any one of the items [1] to [6], in which the component (C) contains a chemical polymerization initiator (C-1).
[8] The dental curable composition according to the item [7], in which the dental curable composition is separately packaged in a first material containing the component (C-1) and a second component containing a polymerization accelerator (F).
[9] The dental curable composition according to the item [7] or [8], in which the first material contains the component (D).
[10] A dental restorative material containing the dental curable composition according to any one of the items [1] to [9].
[11] A dental cement containing the dental curable composition according to any one of the items [1] to [9].
[12] A dental abutment building material containing the dental curable composition according to any one of the items [1] to [9].

### Advantageous Effects of Invention

The present invention can provide a dental curable composition that has an ion releasability, and is excellent in mechanical strength and adhesion durability.

### Description of Embodiments

The present invention will be described in detail with reference to embodiments below.

In the description herein, the upper limit values and the lower limit values of the numeral ranges (such as the contents of the components, the values calculated from the components, and the property values) can be appropriately combined. In the description herein, the values of the symbols in the formulae can also be appropriately combined.

Therefore, in the description herein, the lower limit values and the upper limit values described in a stepwise manner for the numeral ranges can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value. Similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as "10 or more" or "30 or more", and similarly, only the upper limit value can be defined as "90 or less" or "60 or less".

In the description herein, the expression "(meth)acrylic" is used as the concept encompassing both "methacrylic" and "acrylic". This is similarly applied to the analogous expressions including "(meth)acrylate", "(meth)acrylate ester", "(meth)acrylamide", "(meth)acryloyloxy", and the like.

In the description herein, the expression "(meth)acrylic based monomer" or "(meth)acrylic based compound" is used as the concept encompassing both a "(meth)acrylate ester compound" and a "(meth)acrylamide and a derivative thereof".

In the description herein, the expression "(bis)acylphosphine oxide" is used as the concept encompassing both a "bisacylphosphine oxide" and an "acylphosphine oxide".

In the description herein, the "polymerizable monomer" is used as the concept of a (meth)acrylic based compound that is polymerized with the polymerization initiator (C) described later. Therefore, the dental curable composition as one embodiment of the present invention contains a polymerizable monomer that contains a (meth)acrylic based monomer (A) containing two or more (meth)acryloyloxy groups and one or more urethane bond in one molecule, and a (meth)acrylate ester compound (B) containing two or more (meth)acryloyloxy groups and one or more hydroxy group in one molecule.

In the description herein, unless otherwise indicated, the expressions "curability" and "storage stability" mean the "curability" and the "storage stability" of the dental curable composition as one embodiment of the present invention.

In the description herein, unless otherwise indicated, the expression "ion releasability" means the "fluoride ion releasability" and the "calcium ion releasability" of a cured product of the dental curable composition as one embodiment of the present invention.

In the description herein, unless otherwise indicated, the expression "mechanical strength" or "strength" means the "flexural strength" and the "flexural modulus" of a cured product of the dental curable composition as one embodiment of the present invention.

In the description herein, unless otherwise indicated, the expression "adhesion durability" means the "adhesion durability" of a cured product of the dental curable composition as one embodiment of the present invention.

### [Dental Curable Composition]

The dental curable composition as one embodiment of the present invention contains a (meth)acrylic based monomer (A) containing two or more (meth)acryloyloxy groups and one or more urethane bond in one molecule (which may be hereinafter referred simply to as a "component (A)"), a (meth)acrylate ester compound (B) containing two or more (meth)acryloyloxy groups and one or more hydroxy group in one molecule (which may be hereinafter referred simply to as a "component (B)"), a polymerization initiator (C) (which may be hereinafter referred simply to as a "component (C)"), and an ion releasing compound (D) (which may be hereinafter referred simply to as a "component (D)").

The dental curable composition preferably further contains one or more kind selected from the group consisting of a mono(meth)acrylate ester compound (E) that does not contain any of a hydroxy group, an acidic group, an amino group, and an amide group in one molecule (which may be hereinafter referred simply to as a "component (E)"), a polymerization accelerator (F) (which may be hereinafter referred simply to as a "component (F)"), and a filler (G) (which may be hereinafter referred simply to as a "component (G)"), and more preferably further contains the component (E), the component (F), and the component (G).

The components contained in the dental curable composition as one embodiment of the present invention will be described below.

### <(Meth)acrylic based Monomer (A) containing Two or more (Meth)acryloyloxy Groups and One or more Urethane Bond in One Molecule>

The (meth)acrylic based monomer (A) containing two or more (meth)acryloyloxy groups and one or more urethane bond in one molecule is used in the dental curable composition for imparting the strength and the ion releasability to the cured product of the dental curable composition. It is estimated that the urethane bond not only forms a hydrogen bond to exhibit the strength, but also accelerates the propagation of ions through the strong polarity thereof, thereby achieving the ion releasability.

The component (A) can be easily synthesized, for example, by subjecting a compound containing an isocyanate containing an alkylene skeleton or a phenylene skeleton and a (meth)acrylate compound having a hydroxy group (-OH) to addition reaction, and can be easily synthesized, for example, by subjecting a polyol having a polymer skeleton, a compound having an isocyanate group (-NCO), and a (meth)acrylate compound having a hydroxy group (-OH) to addition reaction. Among these, it is preferred that the polymer skeleton is not contained from the standpoint of achieving the excellent strength of the cured product of the dental curable composition.

Examples of the compound having an isocyanate group include methylene diisocyanate (abbr.: MDI), hexamethylene diisocyanate (abbr.: HDI), isophorone diisocyanate (abbr.: IPDI), trimethylhexamethylene diisocyanate (abbr.: TMHMDI), tricyclodecane diisocyanate (abbr.: TCDDI), adamantane diisocyanate (abbr.: ADI), tolylene diisocyanate (abbr.: TDI), xylylene diisocyanate (abbr.: XDI), and diphenylmethane diisocyanate (abbr.: DPMDI). One kind of these compounds may be used alone, or two or more kinds thereof may be used in combination. Among these, IPDI, TMHMDI, and TCDDI are preferred, and TMHMDI is more preferred, from the standpoint of achieving the excellent strength of the cured product of the dental curable composition.

Examples of the (meth)acrylate compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis(4-(3-(meth)acryloyloxy-2-hydroxypropoxy)phenyl)propane, 1,2-bis(3-(meth)acryloyloxy-2-hydroxypropoxy)ethane, pentaerythritol tri(meth)acrylate, and dipentaerythritol tri- or tetra(meth)acrylate. One kind of these compounds may be used alone, or two or more kinds thereof may be used in combination. Among these, 2-hydroxyethyl (meth)acrylate, 2-hydroxypropyl (meth)acrylate, and 2-hydroxy-3-acryloyloxypropyl (meth)acrylate are preferred, and 2-hydroxyethyl (meth)acrylate is more preferred, from the standpoint of achieving the excellent curability of the dental curable composition.

The addition reaction of the compound containing an isocyanate group and the (meth)acrylate compound having a hydroxy group can be performed according to the ordinary method with no particular limitation.

Examples of the component (A) having no polymer skeleton include 2,2,4-trimethylhexamethylenebis(2-carbamoyloxyethyl) dimethacrylate (abbr.: UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propan-1,3-diol] tetramethacrylate (abbr.: U4TH), hexamethylenebis(2-carbamoyloxy-3-phenoxypropyl) diacrylate, and 2,4-tolylenebis(2-carbamoyloxyethyl) hexaacrylate. One kind of these compounds may be used alone, or two or more kinds thereof may be used in combination. Among these, UDMA and U4TH are preferred, and UDMA is more preferred, from the standpoint of enhancing the strength and the ion releasability.

The number of (meth)acryloyloxy groups in one molecule of the component (A) is 2 or more, and is not particularly limited, as far as exhibiting the effects of the present invention, and in one embodiment of the present invention, for example, the number thereof may be 2 to 10, preferably 2 to 6, more preferably 2 to 4, further preferably 2 to 3, and still further preferably 2.

The number of a urethane bond in one molecule of the component (A) is 1 or more, and is not particularly limited, as far as exhibiting the effects of the present invention, and in one embodiment of the present invention, for example, the number thereof may be 1 to 6, preferably 2 to 4, more preferably 2 to 3, and further preferably 2.

The content of the component (A) in the dental curable composition is preferably 20 to 95 parts by mass per 100 parts by mass in total of the polymerizable monomer, and is more preferably 30 to 90 parts by mass, and further preferably 40 to 80 parts by mass, from the standpoint of achieving the excellent ion releasability.

### <(Meth)acrylate Ester Compound (B) containing Two or more (Meth)acryloyloxy Groups and One or more Hydroxy Group in One Molecule>

The (meth)acrylate ester compound (B) containing two or more (meth)acryloyloxy groups and one or more hydroxy group in one molecule is used in the dental curable composition for imparting the adhesiveness to the dental curable composition, and for imparting the ion releasability to the cured product of the dental curable composition.

In the description herein, it is determined that a (meth)acrylate ester compound that corresponds to both the components (A) and (B), i.e., a (meth)acrylate ester compound that contains two or more (meth)acryloyloxy groups in one molecule and contains both one or more urethane bond and one or more hydroxy group in one molecule, is included in the component (A).

Examples of the component (B) include glycerol di(meth)acrylate, 1,2-bis(3-(meth)acryloyloxy-2-hydroxypropoxy)ethane, 2,2-bis(4-(3-(meth)acryloyloxy)-2-hydroxypropoxyphenyl)propane, and pentaerythritol tri(meth)acrylate. One kind of these compounds may be used alone, or two or more kinds thereof may be used in combination. Among these, glycerol di(meth)acrylate and 1,2-bis(3-(meth)acryloyloxy-2-hydroxypropoxy)ethane are preferred, and glycerol dimethacrylate and 1,2-bis(3-methacryloyloxy-2-hydroxypropoxy)ethane are more preferred, from the standpoint of achieving the excellent adhesion durability of the dental curable composition and the excellent effect of enhancing the ion releasability of the cured product.

The number of (meth)acryloyloxy groups in one molecule of the component (B) is 2 or more, and is not particularly limited, as far as exhibiting the effects of the present invention, and in one embodiment of the present invention, for example, the number thereof may be 2 to 10, preferably 2 to 6, more preferably 2 to 4, further preferably 2 to 3, and still further preferably 2.

The number of a hydroxy group in one molecule of the component (B) is 1 or more, and is not particularly limited, as far as exhibiting the effects of the present invention, and in one embodiment of the present invention, for example, the number thereof may be 1 to 4, preferably 1 to 3, and more preferably 1 or 2.

The content of the component (B) in the dental curable composition is preferably 1 to 60 parts by mass per 100 parts by mass in total of the polymerizable monomer, and is more preferably 5 to 50 parts by mass, and further preferably 8 to 40 parts by mass, from the standpoint of achieving the excellent ion releasability of the cured product without impairing the adhesion durability.

The total content of the component (A) and the component (B) in the polymerizable monomer is preferably 30 parts by mass or more, and more preferably 40 parts by mass or more, from the standpoint of further facilitating the effects of the present invention, and is further preferably 50 parts by mass or more, and still further preferably 60 parts by mass or more, from the standpoint of achieving the further excellent ion releasability, per 100 parts by mass in total of the polymerizable monomer. The total content of the component (A) and the component (B) in the polymerizable monomer is 100 parts by mass or less, preferably 98 parts by mass or less, more preferably 96 parts by mass or less, further preferably 94 parts by mass or less, and still further preferably 92 parts by mass or less, per 100 parts by mass in total of the polymerizable monomer, from the standpoint of further facilitating the effects of the present invention. As described above, the lower limit values and the upper limit values described in a stepwise manner can be independently combined. For example, in one embodiment of the present invention, the total content of the component (A) and the component (B) in the polymerizable monomer is preferably 30 to 100 parts by mass, more preferably 40 to 100 parts by mass, further preferably 40 to 98 parts by mass, still further preferably 50 to 96 parts by mass, still more further preferably 60 to 94 parts by mass, and even further preferably 60 to 92 parts by mass, per 100 parts by mass in total of the polymerizable monomer.

### <(Meth)acrylic based monomer containing Two or more (Meth)acryloyloxy Groups in One Molecule other than Components (A) and (B)>

The dental curable composition may further contain a (meth)acrylic based monomer that contains two or more (meth)acryloyloxy groups in one molecule and does not contain any of a urethane bond and a hydroxy group, in addition to the component (A) and the component (B), for the purpose of regulating the strength and the viscosity, and the like, in such a range that does not impair the effects of the present invention. Examples of the (meth)acrylic based monomer that contains two or more (meth)acryloyloxy groups in one molecule and does not contain any of a urethane bond and a hydroxy group include an aromatic compound based (meth)acrylic based monomer that contains two (meth)acryloyloxy group in one molecule and does not contain any of a urethane bond and a hydroxy group; an aliphatic compound based (meth)acrylic based monomer that contains two (meth)acryloyloxy group in one molecule and does not contain any of a urethane bond and a hydroxy group; and a (meth)acrylic based monomer that contains three or more (meth)acryloyloxy group in one molecule and does not contain any of a urethane bond and a hydroxy group.

Examples of the aromatic compound based (meth)acrylic based monomer that contains two (meth)acryloyloxy group in one molecule and does not contain any of a urethane bond and a hydroxy group include 2,2-bis((meth)acryloyloxyphenyl)propane, 2,2-bis(4-(meth)acryloyloxyethoxyphenyl)propane, 2,2-bis(4-(meth)acyrloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxyditriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. One kind of these compounds may be used alone, or two or more kinds thereof may be used in combination.

Examples of the aliphatic compound based (meth)acrylic based monomer that contains two (meth)acryloyloxy group in one molecule and does not contain any of a urethane bond and a hydroxy group include ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, and 1,10-decanediol di(meth)acrylate. One kind of these compounds may be used alone, or two or more kinds thereof may be used in combination.

Examples of the (meth)acrylic based monomer that contains three or more (meth)acryloyloxy group in one molecule and does not contain any of a urethane bond and a hydroxy group include trimethylolpropane tri(meth)acrylate, trimethylolethane tri(meth)acrylate, trimethylolmethane tri(meth)acrylate, pentaerythritol tetra(meth)acrylate, dipentaerythritol penta(meth)acrylate, and 1,7-diacryloyloxy-2,2,6,6-tetraacryloyloxymethyl-4-oxyheptane. The number of the (meth)acryloyl groups in the (meth)acrylic based monomer that contains three or more (meth)acryloyloxy group in one molecule and does not contain any of a urethane bond and a hydroxy group is 3 or more, and is not particularly limited, as far as exerting the effects of the present invention, and in one embodiment of the present invention, the number thereof may be 3 to 10, may be 3 to 8, and may be 3 to 6. One kind of these compounds may be used alone, or two or more kinds thereof may be used in combination.

The content of the (meth)acrylic based monomer that contains two or more (meth)acryloyloxy groups in one molecule and does not contain any of a urethane bond and a hydroxy group other than the components (A) and (B) in the dental curable composition is preferably 40 parts by mass or less per 100 parts by mass in total of the polymerizable monomer, and is more preferably 30 parts by mass or less, further preferably 20 parts by mass or less, and still further preferably 10 parts by mass or less, from the standpoint of achieving the further excellent ion releasability. In other words, in one embodiment of the present invention, the content of the (meth)acrylic based monomer that contains two or more (meth)acryloyloxy groups in one molecule and does not contain any of a urethane bond and a hydroxy group other than the components (A) and (B) is preferably 0 to 40 parts by mass, more preferably 0 to 30 parts by mass, further preferably 0 to 20 parts by mass, and still further preferably 0 to 10 parts by mass, per 100 parts by mass in total of the polymerizable monomer.

### <Mono(meth)acrylate Ester Compound (E) that does not contain any of Hydroxy group, Acidic group, Amino group, and Amide group in One Molecule>

The dental curable composition preferably further contains a mono(meth)acrylate ester compound (E) that does not contain any of a hydroxy group, an acidic group, an amino group, and an amide group in one molecule, as a polymerizable monomer. The component (E) can be preferably used in the dental curable composition from the standpoint of reducing the viscosity of the dental curable composition, enhancing the strength of the cured product, and further imparting the effect of enhancing the adhesion durability with excellent water resistance. Examples of the acidic group include a phosphoric acid group, a sulfo group, and a carboxy group.

Examples of the mono(meth)acrylate ester compound (E) that does not contain any of a hydroxy group, an acidic group, an amino group, and an amide group in one molecule include o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, butoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, 6-(p-phenoxyphenyl)hexyl (meth)acrylate, methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, cyclohexyl (meth)acrylate, 2-ethylhexyl (meth)acrylate, lauryl (meth)acrylate, cetyl (meth)acrylate, stearyl (meth)acrylate, isobornyl (meth)acrylate, benzyl (meth)acrylate, phenyl (meth)acrylate, 2,3-dibromopropyl (meth)acrylate, 3-(meth)acryloyloxypropyltrimethoxysilane, and 11-(meth)acryloyloxyundecyltrimethoxysilane. One kind of these compounds may be used alone, or two or more kinds thereof may be used in combination.

Among these, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, and lauryl methacrylate are preferred, ethoxylated o-phenylphenol (meth)acrylate, ethoxylated m-phenylphenol (meth)acrylate, ethoxylated p-phenylphenol (meth)acrylate, o-phenoxybenzyl (meth)acrylate, m-phenoxybenzyl (meth)acrylate, p-phenoxybenzyl (meth)acrylate, and lauryl methacrylate are more preferred, and ethoxylated o-phenylphenol (meth)acrylate, ethoxylated m-phenylphenol (meth)acrylate, o-phenoxybenzyl (meth)acrylate, and m-phenoxybenzyl (meth)acrylate are further preferred, from the standpoint of achieving the excellent strength and the excellent adhesion durability of the cured product of the dental curable composition. In the preferred embodiments described above, the methacrylate type compounds are still further preferred, and ethoxylated o-phenylphenol methacrylate, ethoxylated m-phenylphenol methacrylate, o-phenoxybenzyl methacrylate, and m-phenoxybenzyl methacrylate are still more further preferred.

In the case where the dental curable composition contains the component (E), the content of the component (E) in the dental curable composition is preferably 5 to 60 parts by mass per 100 parts by mass in total of the polymerizable monomer, and is more preferably 6 to 50 parts by mass, and further preferably 8 to 40 parts by mass, from the standpoint of further facilitating the effects of the present invention.

In the case where the dental curable composition contains the component (E), the total content of the component (A), the component (B), and the component (E) in the polymerizable monomer is preferably 60 to 100 parts by mass per 100 parts by mass in total of the polymerizable monomer, from the standpoint of further facilitating the effects of the present invention, is more preferably 70 to 100 parts by mass, further preferably 80 to 100 parts by mass, and still further preferably 90 to 100 parts by mass, from the standpoint of achieving the further excellent ion releasability, and may be 100 parts by mass.

### <Mono(meth)acrylate Ester Compound other than Component (E)>

The dental curable composition may contain a mono(meth)acrylate ester compound other than the component (E) for the purpose of regulating the adhesiveness, and the like, in such a range that does not impair the effects of the present invention.

Examples of the mono(meth)acrylate ester compound other than the component (E) include 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis(2-(meth)acryloyloxyethyl) hydrogen phosphate, 2-(meth)acryloyloxyethylphenyl hydrogen phosphate, and 2-(meth)acryloyloxyethyl-2-bromoethyl hydrogen phosphate.

The content of the mono(meth)acrylate ester compound other than the component (E) in the dental curable composition is preferably 20 parts by mass or less, more preferably 10 parts by mass or less, and further preferably 5 parts by mass or less, per 100 parts by mass in total of the polymerizable monomer, from the standpoint of facilitating the exhibition of the good adhesion durability. In other words, in one embodiment of the present invention, the content of the mono(meth)acrylate ester compound other than the component (E) is preferably 0 to 20 parts by mass, more preferably 0 to 10 parts by mass, and further preferably 0 to 5 parts by mass, per 100 parts by mass in total of the polymerizable monomer.

In the dental curable composition, the total amount of the polymerizable monomer is preferably 10 to 60% by mass, more preferably 15 to 50% by mass, further preferably 20 to 45 parts by mass, and still further preferably 25 to 40 parts by mass, based on 100% by mass of the dental curable composition, from the standpoint of further facilitating the effects of the present invention.

### <Polymerization Initiator (C)>

The polymerization initiator (C) used may be selected from polymerization initiators that have been used in the general industries, and among these, a polymerization initiator that has been applied to the dental use is preferably used. The polymerization initiator (C) more preferably contains at least one kind selected from the group consisting of a chemical polymerization initiator (C-1) and a photopolymerization initiator (C-2), and further preferably contains at least a chemical polymerization initiator (C-1), and may contain both a chemical polymerization initiator (C-1) and a photopolymerization initiator (C-2).

The chemical polymerization initiator (C-1) used is preferably an organic peroxide. The organic peroxide used as the chemical polymerization initiator (C-1) is not particularly limited, and known compounds can be used. Representative examples of the organic peroxide include a ketone peroxide, a hydroperoxide, a diacyl peroxide, a dialkyl peroxide, a peroxyketal, a peroxyester, and a peroxydicarbonate. One kind of these compounds may be used alone, or two or more kinds thereof may be used in combination.

Examples of the ketone peroxide include methyl ethyl ketone peroxide, methyl isobutyl ketone peroxide, methylcyclohexanone peroxide, and cyclohexanone peroxide.

Examples of the hydroperoxide include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxide include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxide include di-t-butyl peroxide, dicumyl peroxide, t-butyl cumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketal include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of the peroxyester include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydrophthalate, t-butyl peroxy-3,3,5-trimethylhexanoate, t-butyl peroxyacetate, t-butyl peroxybenzoate, and t-butyl peroxymaleate.

Examples of the peroxydicarbonate include di-3-methoxy peroxydicarbonate, di-2-ethylhexyl peroxydicarbonate, bis(4-t-butylcyclohexyl) peroxydicarbonate, diisopropyl peroxydicarbonate, di-n-propyl peroxydicarbonate, di-2-ethoxyethyl peroxydicarbonate, and diallyl peroxydicarbonate.

Among the organic peroxides, the chemical polymerization initiator (C-1) used is more preferably one kind selected from a hydroperoxide and a diacyl peroxide, and further preferably a hydroperoxide, from the standpoint of the overall balance of the safety, the curability, and the storage stability. Among these, the chemical polymerization initiator (C-1) used is preferably one or more kind selected from the group consisting of benzoyl peroxide, cumene hydroperoxide, t-butyl hydroperoxide, and 1,1,3,3-tetramethylbutyl hydroperoxide, more preferably one or more kind selected from the group consisting of cumene hydroperoxide, t-butyl hydroperoxide, and 1,1,3,3-tetramethylbutyl hydroperoxide, and further preferably 1,1,3,3-tetramethylbutyl hydroperoxide.

Examples of the photopolymerization initiator (C-2) include a (bis)acylphosphine oxide compound (including a salt thereof), a thioxanthone compound (including a salt, such as a quaternary ammonium salt, thereof), a ketal compound, an α-diketone compound, a coumarin compound, an anthraquinone compound, a benzoin alkyl ether compound, and an α-aminoketone based compound. One kind of these compounds may be used alone, or two or more kinds thereof may be used in combination.

In the photopolymerization initiator (C-2), at least one kind selected from the group consisting of a (bis)acylphosphine oxide compound and an α-diketone compound is preferably used. According to the configuration, a curable composition that is excellent in photocurability in the visible region and the near ultraviolet regions, and shows sufficient photocurability with the use of any light source including a halogen lamp, a light emitting diode (LED), and a xenon lamp can be obtained.

Examples of the acylphosphine oxide compound in the (bis)acylphosphine oxide compound include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyldi-(2,6-dimethylphenyl) phosphonate, 2,4,6-trimethylbenzoylphenylphosphine oxide sodium salt, 2,4,6-trimethylbenzoylphenylphosphine oxide potassium salt, and 2,4,6-trimethylbenzoylphenylphosphine oxide ammonium salt.

Examples of the bisacylphosphine oxide compound in the (bis)acylphosphine oxide compound include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphosphine oxide, bis(2,4,6-trimethylbenzoyl)phenylphosphine oxide, and bis(2,3,6-trimethylbenzoyl)-2,4,4-trimethylpentylphosphine oxide.

Examples of the (bis)acylphosphine oxide compound also include the compounds described in JP2000-159621A.

Among the (bis)acylphosphine oxide compounds, 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt are more preferably used as the photopolymerization initiator (C-2).

Examples of the α-ketone compound include diacetyl, benzyl, camphorquinone, 2,3-pentadione, 2,3-octadione, 9,10-phenanthrenequinone, 4,4'-oxybenzyl, and acenaphthenequinone. Among these, camphorquinone is preferred from the standpoint of the presence of the maximum absorption wavelength in the visible region.

The content of the component (C) is not particularly limited, and the component (C) is preferably contained in an amount of 0.001 to 30 parts by mass per 100 parts by mass in total of the polymerizable monomer from the standpoint of the curability of the dental curable composition and the like. In the case where the content of the component (C) is 0.001 part by mass or more, the polymerization can be allowed to proceed sufficiently to prevent stickiness. From this standpoint, the content of the component (C) is more preferably 0.03 part by mass or more, further preferably 0.05 part by mass or more, and still further preferably 0.1 part by mass or more, per 100 parts by mass in total of the polymerizable monomer. In the case where the content of the component (C) is 30 parts by mass or less, the deposition of the component (C) from the dental curable composition can be prevented. From this standpoint, the content of the component (C) is more preferably 10 parts by mass or less, further preferably 5 parts by mass or less, and still further preferably 2 parts by mass or less, per 100 parts by mass in total of the polymerizable monomer. As described above, the lower limit values and the upper limit values described in a stepwise manner can be independently combined. For example, in one embodiment of the present invention, the content of the component (C) is preferably 0.03 to 10 parts by mass, more preferably 0.05 to 5 parts by mass, and further preferably 0.1 to 2 parts by mass, per 100 parts by mass in total of the polymerizable monomer.

In the case where the component (C) contains the chemical polymerization initiator (C-1), the content of the chemical polymerization initiator (C-1) is not particularly limited, and in one embodiment of the dental curable composition, the content of the chemical polymerization initiator (C-1) in the component (C) is preferably 60 to 100% by mass, more preferably 70 to 100% by mass, and further preferably 80 to 100% by mass, based on 100% by mass of the component (C), from the standpoint of further facilitating the effects of the present invention.

In the case where the component (C) contains the photopolymerization initiator (C-2), the content of the photopolymerization initiator (C-2) is not particularly limited, and in one embodiment of the dental curable composition, the content of the photopolymerization initiator (C-2) in the component (C) is preferably 1 to 40% by mass, more preferably 5 to 30% by mass, and further preferably 8 to 20% by mass, based on 100% by mass of the component (C), from the standpoint of further facilitating the effects of the present invention.

In one embodiment of the dental curable composition, the total content of the chemical polymerization initiator (C-1) and the photopolymerization initiator (C-2) in the component (C) is preferably 60 to 100% by mass, more preferably 70 to 100% by mass, and further preferably 80 to 100% by mass, and may be 100% by mass, based on 100% by mass of the component (C), from the standpoint of further facilitating the effects of the present invention.

### <Ion Releasing Compound (D)>

The dental curable composition contains an ion releasing compound (D) for the purpose of imparting acid resistance to the tooth substance. Examples of the ion releasing compound (D) include a fluoride ion releasing compound, a calcium ion releasing compound, a zinc ion releasing compound, a strontium ion releasing compound, a phosphate ion releasing compound, and a borate ion releasing compound. Among these, a fluoride ion releasing compound and a calcium ion releasing compound are preferably used due to the large effect of imparting acid resistance to the tooth substance thereof, in which a fluoride ion releasing compound is more preferred. The dental curable composition preferably contains at least one kind selected from the group consisting of a fluoride ion releasing compound and a calcium ion releasing compound, more preferably contains a fluoride ion releasing compound, and further preferably contains both a fluoride ion releasing compound and a calcium ion releasing compound.

Examples of the fluoride ion releasing compound include a fluoride ion releasing polymer, such as a copolymer of a (meth)acrylate ester and a (meth)acrylic acid fluoride; a fluoride ion releasing ammonium salt, such as ammonium hydrofluoride and cetylamine hydrofluoride; a phosphate salt, such as sodium monofluorophosphate; a metal fluoride, such as lithium fluoride, sodium fluoride, potassium fluoride, rubidium fluoride, cesium fluoride, beryllium fluoride, magnesium fluoride, calcium fluoride, strontium fluoride, barium fluoride, aluminum fluoride, manganese(II) fluoride, iron(II) fluoride, iron(III) fluoride, cobalt(II) fluoride, copper(II) fluoride, zinc fluoride, antimony(III) fluoride, lead(II) fluoride, silver(I) fluoride, cadmium fluoride, tin(II) fluoride, tin(IV) fluoride, titanium(IV) fluoride, diammine silver fluoride, sodium hydrogenfluoride, potassium hydrogenfluoride, sodium fluorophosphate, potassium hexafluorotitanate, sodium hexafluorosilicate, sodium hexafluorophosphate, sodium hexafluorostannate(IV), alanine hexafluorostannate(IV), sodium pentafluorodistannate(II), and potassium hexafluorozirconate; and a fluorine-containing glass, such as fluoroaluminosilicate glass, calcium fluoroaluminosilicate glass, zinc fluoroaluminosilicate glass, strontium fluoroaluminosilicate glass, barium fluoroaluminosilicate glass, zinc strontium fluoroaluminosilicate glass, strontium calcium fluoroaluminosilicate glass, fluoroborosilicate glass, fluoroboroaluminosilicate glass, zinc fluoroboroaluminosilicate glass, strontium fluoroboroaluminosilicate glass, and zinc strontium fluoroboroaluminosilicate glass. One kind of these compounds may be used alone, or two or more kinds thereof may be used in combination.

Among these, the fluoride ion releasing compound is preferably sodium fluoride, calcium fluoride, strontium fluoride, zinc fluoride, titanium(IV) fluoride, fluoroaluminosilicate glass, zinc fluoroaluminosilicate glass, strontium fluoroaluminosilicate glass, calcium fluoroaluminosilicate glass, fluoroboroaluminosilicate glass, zinc fluoroboroaluminosilicate glass, strontium fluoroboroaluminosilicate glass, and zinc strontium fluoroboroaluminosilicate glass are preferred, sodium fluoride, calcium fluoride, strontium fluoride, zinc fluoride, titanium(IV) fluoride, fluoroaluminosilicate glass, strontium fluoroboroaluminosilicate glass, and zinc strontium fluoroboroaluminosilicate glass are more preferred, and sodium fluoride, zinc fluoride, and fluoroaluminosilicate glass are further preferred, from the standpoint of facilitating the formation of the acid resistant layer with the excellent ion releasability thereof.

Examples of the calcium ion releasing compound include a calcium salt containing phosphorus, such as calcium silicate glass, tetracalcium phosphate, anhydrous calcium monohydrogen phosphate, tricalcium phosphate, anhydrous calcium dihydrogen phosphate, amorphous calcium phosphate, acidic calcium pyrophosphate, calcium monohydrogen phosphate dihydrate, and calcium dihydrogen phosphate monohydrate; and a calcium salt containing no phosphorus, such as calcium hydroxide, calcium oxide, calcium chloride, calcium nitrate, calcium acetate, calcium lactate, calcium citrate, calcium metasilicate, dicalcium silicate, tricalcium silicate, and calcium carbonate. One kind of these compounds may be used alone, or two or more kinds thereof may be used in combination.

The calcium ion releasing compound is preferably calcium silicate glass, tetracalcium phosphate, anhydrous calcium monohydrogen phosphate, tricalcium phosphate, anhydrous calcium dihydrogen phosphate, amorphous calcium phosphate, calcium hydroxide, calcium oxide, calcium metasilicate, dicalcium silicate, and tricalcium silicate are preferred, and calcium silicate glass, tetracalcium phosphate, anhydrous calcium monohydrogen phosphate, anhydrous calcium dihydrogen phosphate, and amorphous calcium phosphate are more preferred, from the standpoint of facilitating the reinforcement of the tooth substance with the excellent ion releasability thereof.

The component (D) may be used after subjecting to a surface treatment with a known surface treating agent, such as polysiloxane, polyethylene glycol, a fatty acid amide, and a silane coupling agent, in such a range that does not impair the ion releasability, for regulating the miscibility with the component (A) and the component (B). However, it is preferred not to perform the coating treatment that covers the ion releasing compound, such as silica coating, the formation of a core-shell structure, and the like the ion releasability is impaired thereby.

The method of the surface treatment can be performed by the known method with no particular limitation, and examples thereof include a method (1) in which a layer formed of a silanol compound is formed on the surface of the ion releasing compound (D), and then the silanol groups are subjected to intermolecular dehydration condensation; and a method (2) in which a hydrolyzable silane compound is (partially) hydrolyzed to perform intermolecular dehydration condensation of the silanol groups, resulting in an oligomer thereof in advance, the ion releasing compound (D) in the form of powder is added to the siloxane oligomer to form a layer formed of the siloxane oligomer on the surface of the ion releasing compound (D), and then the siloxane oligomer is subjected to intermolecular dehydration condensation to form a polymer.

In the method (1), firstly, a hydrolyzable silane compound and a necessary amount of water are added to a water-miscible organic solvent, such as methanol, ethanol, and a t-butanol, and the hydrolyzable silane compound is (partially) hydrolyzed in the presence of an acid catalyst, so as to prepare an organic solution containing a silanol compound, which is the hydrolysate of the silane compound. The organic solution containing the silanol compound can also be prepared in such a manner that an excessive amount of water is added to a hydrolyzable silane compound, which is (partially) hydrolyzed in the presence of an acid catalyst, so as to prepare an aqueous solution containing a silanol compound, and then the silanol compound in the aqueous solution is extracted with a water-immiscible organic solvent, such as ethyl acetate, ethyl ether, chloroform, and methylene chloride. Subsequently, the ion releasing compound (D) in the form of powder is added to the organic solution obtained by these methods, and then the organic solvent is evaporated off by a heating treatment or a decompression treatment, so as to provide the ion releasing compound (D) in the form of composite particles including the silanol compound attached in the form of a layer on the surface of the ion releasing compound (D). Thereafter, after adding an acid or a base thereto depending on necessity, the silanol groups are subjected to intermolecular dehydration condensation by heating the ion releasing compound (D), resulting in the ion releasing compound (D) in the form of composite particles including a layer formed of polysiloxane formed on the surface of the ion releasing compound (D).

In the method (2), firstly, a prescribed amount of water is added to a hydrolyzable silane compound, which is (partially) hydrolyzed in the presence of an acid catalyst while distilling off the by-produced alcohol, so as to produce a siloxane oligomer. The ion releasing compound (D) in the form of powder is added to the siloxane oligomer to form a layer formed of the siloxane oligomer on the surface of the particles, and after adding an acid or a base thereto depending on necessity, the silanol groups in the siloxane oligomer are subjected to intermolecular dehydration condensation by heating, resulting in the ion releasing compound (D) in the form of composite particles including a layer formed of the polysiloxane formed on the surface of the base particles of the ion releasing compound (D).

The component (D) is not particularly limited in shape, and may be in an optional particle shape, such as a spherical shape, an acicular shape, a planar shape, a fracture shape, and a scale-like shape, and may be dissolved in a polymerizable monomer or the like. In the case where the component (D) is in the form of particles, the average primary particle diameter thereof is preferably 0.001 to 10 µm, more preferably 0.005 to 5.0 µm, further preferably 0.01 to 4.0 µm, and still further preferably 0.05 to 1.0 µm, from the standpoint of the handleability of the dental curable composition, the ion releasability, the mechanical strength, and the transparency of the cured product thereof, and the like. In the description herein, the average primary particle diameter of the component (D) can be obtained through observation with an optical microscope or an electron microscope. Specifically, observation with an optical microscope is convenient for measuring the particle diameter of 100 nm or more, and observation with an electron microscope is convenient for measuring the particle diameter of less than 100 nm.

The content of the component (D) is not particularly limited, and is preferably 1.0 to 400 parts by mass, more preferably 10 to 300 parts by mass, and further preferably 20 to 200 parts by mass, per 100 parts by mass in total of the polymerizable monomer, from the standpoint of the handleability of the resulting composition and the ion releasability of the cured product thereof.

The total amount of the component (A), the component (B), the component (C), and the component (D) in the dental curable composition is not particularly limited, as far as exhibiting the effects of the present invention, and is preferably 30% by mass or more, more preferably 35% by mass or more, and further preferably 40% by mass or more, and is 100% by mass or less, preferably 80% by mass or less, more preferably 75% by mass or less, and further preferably 70% by mass or less, based on 100% by mass of the dental curable composition, from the standpoint of further facilitating the effects of the present invention. As described above, the lower limit values and the upper limit values described in a stepwise manner can be independently combined. For example, in one embodiment of the present invention, the total amount of the component (A), the component (B), the component (C), and the component (D) in the dental curable composition is preferably 30 to 100% by mass, more preferably 30 to 80% by mass, further preferably 35 to 75% by mass, and still further preferably 40 to 70% by mass, based on 100% by mass of the dental curable composition.

### <Polymerization Accelerator (F)>

The dental curable composition preferably further contains a polymerization accelerator (F). Examples of the polymerization accelerator (F) include an amine compound, sulfinic acid and a salt thereof, a sulfite salt, a bisulfite salt, an aldehyde compound, a thiourea compound, an organic phosphorus compound, a borate compound, barbituric acid compound and a derivative thereof, a triazine compound, a vanadium compound, a copper compound, a tin compound, a cobalt compound, a halogen compound, and a thiol compound. One kind of these compounds may be used alone, or two or more kinds thereof may be used in combination.

The amine compound is classified into an aliphatic amine and an aromatic amine.

Examples of the aliphatic amine include a primary aliphatic amine, such as n-butylamine, n-hexylamine, and n-octylamine; a secondary aliphatic amine, such as diisopropylamine, dibutylamine, and N-methylethanolamine; and a tertiary aliphatic amine, such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, trimethylamine, triethylamine, and tributylamine.

Examples of the aromatic amine include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate.

Among these, at least one kind selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone is preferably used from the standpoint of imparting the excellent curability to the dental curable composition.

Examples of the sulfinic acid and a salt thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate.

Examples of the sulfite salt and the bisulfite salt include sodium sulfite, potassium sulfite, calcium sulfite, ammonium sulfite, sodium bisulfite, and potassium bisulfite.

Examples of the aldehyde compound include terephthalaldehyde and a benzaldehyde derivative. Examples of the benzaldehyde derivative include dimethylaminobenzaldehyde, p-methyloxybenzaldehyde, p-ethyloxybenzaldehyde, and p-n-octyloxybenzaldehyde.

Examples of the thiourea compound include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 3,3-dimethylethylenethiourea, and 4,4-dimethylethylenethiourea. Among these, at least one kind selected from the group consisting of 1-(2-pyridyl)-2-thiourea and 4,4-dimethylethylenethiourea is preferably used from the standpoint of the curability of the dental curable composition.

Examples of the organic phosphorus compound include triphenylphosphine, 2-methyltriphenylphosphine, 4-methyltriphenylphosphine, 2-methoxytriphenylphosphine, 4-methoxytriphenylphosphine, tri-n-butylphosphine, triisobutylphosphine, and tri-t-butylphosphine.

The borate compound is preferably an arylborate compound. As specific examples of the arylborate compound that can be preferably used, examples of the borate compound having one aryl group in one molecule include a trialkylphenylboron, a trialkyl(p-chlorophenyl)boron, a trialkyl(p-fluorophenyl)boron, a trialkyl(3,5-bis(trifluoromethyl)phenyl)boron, a trialkyl(3,5-bis(1,1,1,3,3,3-hexafluoro-2-methoxy-2-propyl)phenyl)boron, a trialkyl(p-nitrophenyl)boron, a trialkyl(m-nitrophenyl)boron, a trialkyl(p-butylphenyl)boron, a trialkyl(m-butylphenyl)boron, a trialkyl(p-butyloxyphenyl)boron, a trialkyl(m-butyloxyphenyl)boron, a trialkyl(p-octyloxyphenyl)boron, and a trialkyl(m-octyloxyphenyl)boron (in which the alkyl group is at least one kind selected from the group consisting of a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having two aryl groups in one molecule include a dialkyldiphenylboron, a dialkyldi(p-chlorophenyl)boron, a dialkyldi(p-fluorophenyl)boron, a dialkyldi(3,5-bis(trifluoromethyl)phenyl)boron, a dialkyldi(3,5-bis(1,1,1,3,3,3-hexafluoro-2-methoxy-2-propyl)phenyl)boron, a dialkyldi(p-nitrophenyl)boron, a dialkyldi(m-nitrophenyl)boron, a dialkyldi(p-butylphenyl)boron, a dialkyldi(m-butylphenyl)boron, a dialkyldi(p-butyloxyphenyl)boron, a dialkyldi(m-butyloxyphenyl)boron, a dialkyldi(p-octyloxyphenyl)boron, and a dialkyldi(m-octyloxyphenyl)boron (in which the alkyl group is at least one kind selected from the group consisting of a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having three aryl groups in one molecule include a monoalkyltriphenylboron, a monoalkyltri(p-chlorophenyl)boron, a monoalkyltri(p-fluorophenyl)boron, a monoalkyltri(3,5-bis(trifluoromethyl)phenyl)boron, a monoalkyltri(3,5-bis(1,1,1,3,3,3-hexafluoro-2-methoxy-2-propyl)phenyl)boron, a monoalkyltri(p-nitrophenyl)boron, a monoalkyltri(m-nitrophenyl)boron, a monoalkyltri(p-butylphenyl)boron, a monoalkyltri(m-butylphenyl)boron, a monoalkyltri(p-butyloxyphenyl)boron, a monoalkyltri(m-butyloxyphenyl)boron, a monoalkyltri(p-octyloxyphenyl)boron, and a monoalkyltri(m-octyloxyphenyl)boron (in which the alkyl group is one kind selected from a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having four aryl groups in one molecule include tetraphenylboron, tetrakis(p-chlorophenyl)boron, tetrakis(p-fluorophenyl)boron, tetrakis(3,5-bis(trifluoromethyl)phenyl)boron, tetrakis(3,5-bis(1,1,1,3,3,3-hexafluoro-2-methoxy-2-propyl)phenyl)boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, (3,5-bis(trifluoromethyl)phenyl)triphenylboron, (p-nitrophenyl)triphenylboron, (m-butyloxyphenyl)triphenylboron, (p-butyloxyphenyl)triphenylboron, (m-octyloxyphenyl)triphenylboron, (p-octyloxyphenyl)triphenylboron, and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Among these arylborate compounds, a borate compound having three or four aryl groups in one molecule is more preferably used from the standpoint of the storage stability. One kind of the arylborate compound may be used alone, or two or more kinds thereof may be used as a mixture.

Examples of the barbituric acid compound and a derivative thereof include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, a thiobarbituric acid compound, and salts thereof (in which an alkali metal salt and an alkaline earth metal salt are preferred). Examples of the salt of the barbituric acid compound include sodium 5-butylbarbiturate, sodium 1,3,5-trimethylbarbiturate, and sodium 1-cyclohexyl-5-ethylbarbiturate.

Examples of the triazine compound include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s-triazine, 2-(2-(p-methoxyphenyl)ethenyl)-4,6-bis(trichloromethyl)-s-triazine, 2-(2-(o-methoxyphenyl)ethenyl)-4,6-bis(trichloromethyl)-s-triazine, 2-(2-(p-butoxyphenyl)ethenyl)-4,6-bis(trichloromethyl)-s-triazine, 2-(2-(3,4-dimethoxyphenyl)ethenyl)-4,6-bis(trichloromethyl)-s-triazine, 2-(2-(3,4,5-trimethoxyphenyl)ethenyl)-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-(2-(N,N-bis(2-hydroxyethyl)amino)ethoxy)-4,6-bis(trichloromethyl)-s-triazine, 2-(2-(N-hydroxyethyl-N-ethylamino)ethoxy)-4,6-bis(trichloromethyl)-s-triazine, 2-(2-(N-hydroxyethyl-N-methylamino)ethoxy)-4,6-bis(trichloromethyl)-s-triazine, and 2-(2-(N,N-diallylamino)ethoxy)-4,6-bis(trichloromethyl)-s-triazine.

The vanadium compound is preferably a tetravalent and/or pentavalent vanadium compound. Examples of the tetravalent and/or pentavalent vanadium compound include divanadium(IV) tetroxide, vanadyl(IV) acetylacetonate, vanadyl(IV) oxalate, vanadyl(IV) sulfate, vanadium(IV) oxobis(1-phenyl-1,3-butanedionate), oxovanadium(IV) bis(maltolate), vanadium(V) pentoxide, sodium metavanadate(V), and ammonium metavanadate(V). Among these, vanadyl(IV) acetylacetonate is preferably used from the standpoint of the curability of the dental curable composition.

Examples of the copper compound include copper acetylacetonate, cupric acetate, copper oleate, cupric chloride, and cupric bromide. Among these, at least one kind selected from the group consisting of copper acetylacetonate and cupric acetate is preferably used from the standpoint of the curability of the dental curable composition.

Examples of the tin compound include di-n-butyltin dimaleate, di-n-octyltin dimaleate, di-n-octyltin dilaurate, and di-n-butyltin dilaurate. Among these, at least one kind selected from the group consisting of di-n-octyltin dilaurate and di-n-butyltin dilaurate is preferably used.

Examples of the cobalt compound include cobalt acetylacetonate, cobalt acetate, cobalt oleate, cobalt chloride, and cobalt bromide.

Examples of the halogen compound that is preferably used include dilauryldimethylammonium chloride, lauryldimethylbenzylammonium chloride, benzyltrimethylammonium chloride, tetramethylammonium chloride, benzyldimethylcetylammonium chloride, and dilauryldimethylammonium chloride.

Examples of the thiol compound include 3-mercaptopropyltrimethoxysilane, 2-mercaptobenzoxazole, decanethiol, and thiobenzoic acid.

Among the polymerization accelerators (F), at least one kind selected from the group consisting of an amine compound, a thiourea compound, a vanadium compound, and a copper compound is more preferably used, and among these, at least one kind selected from the group consisting of 1-(2-pyridyl)-2-thiourea, N,N-di(2-hydroxyethyl)-p-toluidine, ethyl 4-(N,N-dimethylamino)benzoate, 4,4-dimethylethylenethiourea, vanadyl(IV) acetylacetonate, copper acetylacetonate, and cupric acetate is further preferably used as the component (F).

The content of the component (F) in the dental curable composition is not particularly limited, and the content of the component (F) is preferably 0.001 to 30 parts by mass per 100 parts by mass in total of the polymerizable monomer from the standpoint of the curability of the dental curable composition, and the like. In the case where the content of the polymerization accelerator (F) is 0.001 part by mass or more, the polymerization can be allowed to proceed sufficiently to prevent stickiness. From this standpoint, the content of the component (F) is more preferably 0.05 part by mass or more, further preferably 0.1 part by mass or more, and still further preferably 0.3 part by mass or more, per 100 parts by mass in total of the polymerizable monomer. In the case where the content of the component (F) is 30 parts by mass or less, the deposition of the component (F) from the dental curable composition can be prevented. From this standpoint, the content of the component (F) is more preferably 20 parts by mass or less, more preferably 10 parts by mass or less, and further preferably 5.0 parts by mass or less, per 100 parts by mass in total of the polymerizable monomer. As described above, the lower limit values and the upper limit values described in a stepwise manner can be independently combined. For example, in one embodiment of the present invention, the content of the component (F) is more preferably 0.05 to 20 parts by mass, further preferably 0.1 to 10 parts by mass, and still further preferably 0.3 to 5.0 parts by mass, per 100 parts by mass in total of the polymerizable monomer.

In the present invention, the chemical polymerization initiator (C-1) and the polymerization accelerator (F) can be used in combination as a redox polymerization initiator. In this case, the chemical polymerization initiator (C-1) and the polymerization accelerator (F) are preferably separately packaged in separate containers from the standpoint of the storage stability. In this case, the dental curable composition is preferably provided as the dental curable composition that is separately packaged at least in the first material containing the chemical polymerization initiator (C-1) and the second component containing the polymerization accelerator (F).

The dental curable composition is more preferably provided as a kit used in the form of two-component system including the first material and the second material. In this case, the dental curable composition is further preferably provided as a kit used in the form of twopaste system, in which both the first material and the second material are in the form of paste. In the case where the dental curable composition is provided as a kit used in the form of twopaste system, the pastes are stored in such a manner that the pastes are isolated from each other, and immediately before use, the two pastes are kneaded to perform chemical polymerization. In the case where the dental curable composition further contains the photopolymerization initiator (C-2), it is preferred that photopolymerization is performed in addition to the chemical polymerization, and thereby the dental curable composition is cured.

In one embodiment of the dental curable composition that is separately packaged in the first material and the second material, it is more preferred that the first material further contains the component (D), and it is further preferred that the first material further contains both the component (B) and the component (D), from the standpoint of achieving the further excellent ion releasability of the cured product of the dental curable composition.

### <Filler (G)>

The dental curable composition preferably further contains a filler (G) from the standpoint of regulating the paste property of the dental curable composition before curing, imparting X-ray radiopacity to the cured product, or further enhancing the strength of the cured product, and the like. Examples of the filler that can be used as the component (G) include an organic filler, an inorganic filler, and an organic-inorganic composite filler.

Examples of the material of the organic filler include polymethyl methacrylate, polyethyl methacrylate, a methyl methacrylate-ethyl methacrylate copolymer, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. One kind of these materials may be used alone, or two or more kinds thereof may be used as a mixture. The shape of the organic filler is not particularly limited, and can be appropriately selected from an irregularly shaped filler, a spherical filler, and the like, and the particle diameter of the filler can be appropriately selected in use.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, and strontium boroaluminosilicate glass. One kind of these materials may be used alone, or two or more kinds thereof may be used as a mixture. The shape of the inorganic filler is not particularly limited, and can be appropriately selected from an irregularly shaped filler, a spherical filler, and the like, and the particle diameter of the filler can be appropriately selected in use.

The inorganic filler may be subjected to a surface treatment with a known surface treating agent, such as a silane coupling agent, in advance depending on necessity for regulating the miscibility thereof with the component (A) and the component (B). Examples of the surface treating agent include vinyltrimethoxysilane, vinyltriethoxysilane, vinyltrichlorosilane, vinyltri(β-methoxyethoxy)silane, 3-methacryloyloxypropyltrimethoxysilane, 11-methacryloyloxyundecyltrimethoxysilane, 3-glycidoxypropyltrimethoxysilane, 3-mercaptopropyltrimethoxysilane, and 3-aminopropyltriethoxysilane.

The method of the surface treatment used may be any known method with no particular limitation, and examples thereof include a method of spraying the surface treating agent on the inorganic filler under vigorous agitation, a method of dispersing or dissolving the inorganic filler and the surface treating agent in a suitable solvent, from which the solvent is then removed, and a method of converting the alkoxy group of the surface treating agent to a silanol group through hydrolysis in an aqueous solution, and attaching the surface treating agent to the surface of the inorganic filler in the aqueous solution, from which water is then removed. In all the methods, the surface treatment can be performed by completing the reaction between the surface of the inorganic filler and the surface treating agent by heating generally to a range of 50 to 150°C.

The organic-inorganic composite filler can be obtained in such a manner that a monomer compound is added to the inorganic filler in advance, so as to make a paste, which is then polymerized and pulverized. Examples of the organic-inorganic composite filler used include a TMPT filler (which is obtained in such a manner that trimethylolpropane trimethacrylate and a silica filler are mixed, polymerized, and then pulverized). The shape of the organic-inorganic composite filler is not particularly limited, and can be appropriately selected from an irregularly shaped filler, a spherical filler, and the like, and the particle diameter of the filler can be appropriately selected in use.

The average primary particle diameter of the component (G) is preferably 0.001 to 10 µm, more preferably 0.005 to 5.0 µm, further preferably 0.01 to 4.0 µm, and still further preferably 0.04 to 3.0 µm, from the standpoint of the handleability of the dental curable composition, the mechanical strength and the transparency of the cured product thereof, and the like. In the description herein, the average primary particle diameter of the component (G) can be obtained through observation with an optical microscope or an electron microscope. Specifically, observation with an optical microscope is convenient for measuring the particle diameter of 100 nm or more, and observation with an electron microscope is convenient for measuring the particle diameter of less than 100 nm.

The content of the component (G) that has an average primary particle diameter of 0.01 µm or more is preferably 80 parts by mass or more, more preferably 90 parts by mass or more, further preferably 95 parts by mass or more, and still further preferably 98 parts by mass or more, per 100 parts by mass in total of the component (G), from the standpoint of imparting X-ray radiopacity to the cured product of the dental curable composition, further enhancing the mechanical strength of the cured product, and the like. In other words, in one embodiment of the present invention, the content of the component (G) that has an average primary particle diameter of 0.01 µm or more is preferably 80 to 100 parts by mass, more preferably 90 to 100 parts by mass, further preferably 95 to 100 parts by mass, and still further preferably 98 to 100 parts by mass, per 100 parts by mass in total of the component (G).

It is further preferred that the dental curable composition contains both the component (G) that has an average primary particle diameter of 0.01 µm or more and the component (G) that has an average primary particle diameter of less than 0.01 µm.

The content of the component (G) is not particularly limited, and is preferably 50 to 500 parts by mass, more preferably 60 to 400 parts by mass, further preferably 65 to 300 parts by mass, and still further preferably 70 to 200 parts by mass, per 100 parts by mass in total of the polymerizable monomer, from the standpoint of the handleability of the dental curable composition and the strength of the cured product thereof.

In the case where the dental curable composition contains the component (E), the component (F), and the component (G), in addition to the components (A) to (D), the total amount of the components (A) to (G) in the dental curable composition is not particularly limited, as far as exhibiting the effects of the present invention, is preferably 80 to 100% by mass, and more preferably 85 to 100% by mass, from the standpoint of further facilitating the effects of the present invention, and is further preferably 90 to 100% by mass, still further preferably 95 to 100% by mass, and still more further preferably 98 to 100% by mass, from the standpoint of achieving the further excellent ion releasability, all based on 100% by mass of the dental curable composition.

The total amount of the polymerizable monomer, the component (C), the component (D), the component (F), and the component (G) in the dental curable composition is not particularly limited, as far as exhibiting the effects of the present invention, and is preferably 80 to 100% by mass, more preferably 85 to 100% by mass, further preferably 90 to 100% by mass, still further preferably 95 to 100% by mass, and still more further preferably 98 to 100% by mass, based on 100% by mass of the dental curable composition, from the standpoint of further facilitating the effects of the present invention.

### <Additional Additives>

The dental curable composition may contain known additional additives depending on necessity in such a range in which the effects of the present invention can be exerted. Examples of the additional additives include components other than described above, such as a polymerization inhibitor, an antioxidant, a pigment, a dye, an ultraviolet ray absorbent, an organic solvent, and a thickener. In the description herein, "the additional additives" do not include a polymerizable monomer.

Examples of the polymerization inhibitor include hydroquinone, hydroquinone monomethyl ether, dibutyl hydroquinone, dibutyl hydroquinone monomethyl ether, t-butyl catechol, 2-t-butyl-4,6-dimethylphenol, 2,6-di-t-butylphenol, and 3,5-di-t-butyl-4-hydroxytoluene. In the case where the dental curable composition contains the polymerization inhibitor, the content of the polymerization inhibitor is preferably 0.001 to 1.0 part by mass, more preferably 0.005 to 0.5 part by mass, and further preferably 0.01 to 0.1 part by mass, per 100 parts by mass in total of the polymerizable monomer.

The total amount of the additional additives in the dental curable composition is not particularly limited, as far as exhibiting the effects of the present invention, is preferably 20% by mass or less, more preferably 15% by mass or less, further preferably 10% by mass or less, still further preferably 5% by mass or less, and still more further preferably 2% by mass or less, and is 0% by mass or more, based on 100% by mass of the dental curable composition, from the standpoint of further facilitating the effects of the present invention. In other words, in one embodiment of the present invention, the total amount of the additional additives in the dental curable composition is preferably 0 to 20% by mass, more preferably 0 to 15% by mass, further preferably 0 to 10% by mass, still further preferably 0 to 5% by mass, and still more further preferably 0 to 2% by mass, based on 100% by mass of the dental curable composition.

The dental curable composition is not particularly limited, as far as containing the component (A), the component (B), the component (C), and the component (D), and preferably contains substantially no polycarboxylic acid from the standpoint of preventing the deterioration of the strength and the water resistance of the cured product, and preventing white turbidity and the like thereof. The fact that substantially no polycarboxylic acid is contained means that the content of a polycarboxylic acid is 5.0% by mass or less, preferably 1.0% by mass or less, more preferably 0.5% by mass or less, further preferably 0.1% by mass or less, and still further preferably 0.05% by mass or less, based on 100% by mass of the dental curable composition. In other words, the fact that substantially no polycarboxylic acid is contained means that the content of a polycarboxylic acid is 0 to 5.0% by mass, preferably 0 to 1.0% by mass, more preferably 0 to 0.5% by mass, further preferably 0 to 0.1% by mass, still further preferably 0 to 0.05% by mass, based on 100% by mass of the dental curable composition.

The dental curable composition preferably contains substantially no water from the standpoint of further improving the strength and the color tone of the resulting cured product. The fact that substantially no water is contained means that the content of water is 5.0% by mass or less, preferably 1.0% by mass or less, more preferably 0.5% by mass or less, further preferably 0.1% by mass or less, and still further preferably 0.05% by mass or less, based on 100% by mass of the dental curable composition. In other words, the fact that substantially no water is contained means that the content of water is 0 to 5.0% by mass, preferably 0 to 1.0% by mass, more preferably 0 to 0.5% by mass, further preferably 0 to 0.1% by mass, still further preferably 0 to 0.05% by mass, based on 100% by mass of the dental curable composition.

The dental curable composition is excellent in ion releasability, mechanical strength, and adhesion durability of the cured product. Therefore, the dental curable composition can be favorably applied to a dental composite resin that can utilize the advantages thereof, and in particular, can be optimally applied to a dental filling and restorative material, a dental cement, and a dental abutment building material.

The dental curable composition is not particularly limited in the mixing method of the components, and the like, except for the matters described for the use in the form of two-component system including the first material and the second material, and can be produced according to a method known by a skilled person in the art.

### Examples

The present invention will be described more specifically with reference to examples below, but the present invention is not limited to the examples.

The components used in Examples and Comparative Examples and abbreviated names thereof will be described below.

### [(Meth)acrylic based Monomer (A) containing Two or more (Meth)acryloyloxy Groups and One or more Urethane Bond in One Molecule]

UDMA: 2,2,4-trimethylhexamethylenebis(2-carbamoyloxyethyl) dimethacrylate (available from Kyoeisha Chemical Co., Ltd.)
U4TH: N,N'-(2,2,4-trimethylhexamethylene)bis[2-(aminocarboxy)propan-1,3-diol] tetramethacrylate (available from Kyoeisha Chemical Co., Ltd.)

### [(Meth)acrylate Ester Compound (B) containing Two or more (Meth)acryloyloxy Groups and One or more Hydroxy Group in One Molecule]

BMHPE: 1,2-bis(3-methacryloyloxy-2-hydroxypropoxy)ethane (available from Shin-Nakamura Chemical Co., Ltd.)
GDM: glycerol dimethacrylate (available from Shin-Nakamura Chemical Co., Ltd.)

### [Mono(meth)acrylate Ester Compound (E) that does not contain any of Hydroxy group, Acidic group, Amino group, and Amide group in One Molecule]

POBMA: m-phenoxybenzyl methacrylate (available from Kyoeisha Chemical Co., Ltd.)
LMA: lauryl methacrylate (available from Kyoeisha Chemical Co., Ltd.)

### [(Meth)acrylic based monomer containing Two or more (Meth)acryloyloxy Groups in One Molecule other than Components (A) and (B)]

D2.6E: 2,2-bis(4-methacryloyloxypolyethoxyphenyl)propane (average addition number of ethoxy group: 2.6, available from Shin-Nakamura Chemical Co., Ltd.)

### [Mono(meth)acrylate Ester Compound other than Component (E)]

HEMA: 2-hydroxyethyl methacrylate

### [Polymerization Initiator (C)]

### <Chemical Polymerization Initiator (C-1)>

THP: 1,1,3,3-tetramethylbutyl hydroperoxide
BPO: benzoyl peroxide

### <Photopolymerization Initiator (C-2)>

CQ: camphorquinone

### [Ion Releasing Compound (D)]

Compound (D-1): 3-methacryloyloxypropyltrimethoxysilane-treated fluoroaluminosilicate glass "SCHOTT (registered trademark) G018-090 UF0.7" (available from SCHOTT AG, average primary particle diameter: 0.7 µm)
Compound (D-2): sodium fluoride (available from Fujifilm Wako Pure Chemical Corporation, average primary particle diameter: 2.5 µm)
Compound (D-3): tetracalcium phosphate (available from Taihei Chemical Industrial Co., Ltd., average primary particle diameter: 3.2 µm)

### [Polymerization Accelerator (F)]

PTU: 1-(2-pyridyl)-2-thiourea
VOAA: vanadyl acetylacetonate
DEPT: N,N-di(2-hydroxyethyl)-p-toluidine
PDE: ethyl 4-N,N-dimethylaminobenzoate

### [Filler (G)]

Filler (G-1): 3-methacryloyloxypropyltrimethoxysilane-treated barium glass "SCHOTT (registered trademark) 8235 UF2.0" (available from SCHOTT AG, average primary particle diameter: 2.0 µm)
Filler (G-2): hydrophilic fumed silica " AEROSIL (registered trademark) 380" (available from Nippon Aerosil Co., Ltd., average primary particle diameter: 0.007 µm)

### [Polymerization Inhibitor]

BHT: 3,5-di-t-butyl-4-hydroxytoluene

### [Examples 1 to 12 and Comparative Example 1 to 3]

The components shown in Tables 1 to 3 below were mixed at ordinary temperature (25°C) to prepare pastes of the first material and the second material, resulting in the dental curable compositions of Examples and Comparative Examples. A kneading apparatus used included a polyolefin based resin container including one pair of barrels having the same shape which are arranged in parallel (total capacity: 5 mL, available from MixPac AG, product code: SDL 005-01-SI), a mixer attached to the top end of the container, and an extruding device including one pair of cylindrical extruding members fixed to each other inserted to the container from the rear end of the container (available from MixPac AG, product code: PED 005-01-SI). The same amounts of the pastes were collected by pressing the extruding members. The mixer used included a mixing chip having eight agitation blades (elements) (available from MixPac AG, product code: ML 2.5-08-S").

The resulting dental curable compositions were measured and evaluated for the fluoride ion and calcium ion releasability, the flexural strength, the flexural modulus, and the adhesion durability in the following manner.

### <Preparation Example 1>

A tooth substance adhesion primer having the following composition was prepared.

Tooth substance adhesion primer:
MDP: 10-methacryloyloxydecyl dihydrogen phosphate 10 parts by mass
HEMA: 2-hydroxyethyl methacrylate 25 parts by mass
DMAEMA: N,N-dimethylaminoethyl methacrylate 3.0 parts by mass
Purified water: 60 parts by mass
VOAA: 0.5 part by mass
BHT: 1.5 parts by mass

### <Fluoride Ion Releasability>

A kneaded product of each of the dental curable compositions of Examples and Comparative Examples was charged in a metal mold having a diameter of 15 mm and a thickness of 1 mm, which was allowed to stand in a thermostat chamber at 37°C for 1 hour for curing, and then the cured product was taken out from the metal mold and immersed in 4 mL of a 0.2 M phosphate buffer solution (pH 7, 37°C). After immersing for 28 days, 2 mL of a TISAB solution was added thereto, and fluoride ion eluted to the phosphate buffer solution was quantitatively determined with a fluoride ion electrode (available from Thermo Fisher Scientific, Inc.). In this test, 200 µg/g or more shows a tendency that an acid resistant layer is formed, showing a good fluoride ion releasability.

### <Calcium Ion Releasability>

A kneaded product of each of the dental curable compositions of Examples and Comparative Examples was charged in a metal mold having a diameter of 15 mm and a thickness of 1 mm, which was allowed to stand in a thermostat chamber at 37°C for 1 hour for curing, and then the cured product was taken out from the metal mold and immersed in 5 mL of ion exchanged water (37°C). After immersing for 28 days, 5 mL of a KCl aqueous solution (15 g/L) was added thereto, and calcium ion eluted to the ion exchanged water was quantitatively determined with a calcium ion electrode (available from Horiba, Ltd.). In this test, 100 µg/g or more shows a tendency that an acid resistant layer is formed, showing a good calcium ion releasability.

### <Flexural Strength and Flexural modulus>

Evaluation was performed by a flexural test according to ISO 4049. A kneaded product of each of the dental curable compositions of Examples and Comparative Examples was charged in a stainless steel mold (2 mm in vertical length × 2 mm in thickness × 25 mm in horizontal length), which was held in the vertical direction with slide glass under pressure, and the dental curable composition was cured by immersing in a thermostat water bath at 37°C. The resulting cured product was subjected to a flexural test by using a universal testing machine ("Autograph (registered trademark) AG-I 100kN", available from Shimadzu Corporation) at a crosshead speed of 2 mm/min, so as to measure the flexural strength and the flexural modulus. In this test, the flexural strength is preferably 100 MPa or more, and more preferably 120 MPa or more, and the flexural modulus is preferably 5.0 GPa or more, and more preferably 6.0 GPa or more.

### <Adhesion Durability>

The labial surface of a bovine mandibular incisor was polished with silicon carbide paper #80 (available from Nihon Kenshi Co., Ltd.) under running water, so as to form a flat surface of the dentin. The flat surface was further polished with silicon carbide paper #1000 (available from Nihon Kenshi Co., Ltd.) under running water, so as to form a flat smooth surface. A surgical tape having an approximately 1 cm square shape with a circular hole having a diameter of 3.0 mm and a depth of 0.2 mm was attached to the flat smooth surface, so as to determine the adhesion area. The tooth substance adhesion primer prepared above was coated inside the circular hole with a brush, and after allowing to stand for 20 seconds, the surface was air-blown to dry until the coated tooth substance adhesion primer lost fluidity.

Subsequently, a commercially available metal adhesion primer (Alloy Primer, available from Kuraray Noritake Dental, Inc.) was coated on one end surface (circular cross sectional surface) of a stainless steel round bar (diameter: 7 mm, length: 2.5 cm), and after evaporating the solvent, a kneaded product of the dental curable composition was coated thereon. The stainless steel round bar was held upright and adhered thereon in such a manner that the coated kneaded product of the dental curable composition was charged in the circular hole. After adhering, the assembly specimen was allowed to stand at room temperature for 30 minutes, and then immersed in distilled water. Ten specimens were produced for the adhesion test, and all the specimens immersed in distilled water were stored in a thermostat chamber held at 37°C. After 24 hours, the specimen was taken out from water, and measured for the tensile adhesion strength with a universal tester (available from Shimadzu Corporation). The tensile adhesion strength was measured with a crosshead speed of 2 mm/min. The average value of the measured values of five specimens was designated as the tensile adhesion strength. In Tables 1 to 3 below, the results are shown as "Adhesion strength after 24 hours".

The remaining five specimens obtained by adhering to the dentin each were subjected to a thermal cycle load of immersing in a water tank at 4°C and a water tank at 60°C alternately each for 1 minute 4,000 times, and then measured for the tensile adhesion strength. The adhesion durability was evaluated by the tensile adhesion strength after the thermal cycle load. In Tables 1 to 3 below, the results are shown as "Adhesion strength after TC4000".

In this test, a specimen having an adhesion strength after 24 hours of 10 MPa or more and an adhesion strength after the thermal cycle test 4,000 times of 8.0 MPa or more was evaluated as having excellent adhesion durability.

**Table 1**

| | | | | Example 1 | | | Example 2 | | | Example 3 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 50 | 80 | 65 | 40 | 50 | 45 | 60 | 90 | 75 |
| | | U4TH | (part by mass) | | | | | | | | | |
| | (B) | BMHPE | (part by mass) | 30 | | 15 | 30 | 10 | 20 | 20 | | 10 |
| | | GDM | (part by mass) | | | | | | | | | |
| | (E) | POBMA | (part by mass) | 20 | 20 | 20 | 30 | 40 | 35 | 20 | 10 | 15 |
| | | LMA | (part by mass) | | | | | | | | | |
| | (C) | THP | (part by mass) | 2.0 | | 1.0 | 2.0 | | 1.0 | 2.0 | | 1.0 |
| | | BPO | (part by mass) | | | | | | | | | |
| | | CQ | (part by mass) | | 0.2 | 0.1 | | 0.2 | 0.1 | | | |
| | (D) | Compound (D-1) | (part by mass) | 200 | | 100 | 250 | | 125 | 150 | | 75 |
| Material | | Compound (D-2) | (part by mass) | | | | | | | | | |
| | | Compound (D-3) | (part by mass) | 5.0 | | 2.5 | 5.0 | | 2.5 | 5.0 | | 2.5 |
| | (F) | PTU | (part by mass) | | 1.5 | 0.75 | | 1.5 | 0.75 | | 1.5 | 0.75 |
| | | VOAA | (part by mass) | | 0.05 | 0.025 | | 0.05 | 0.025 | | 0.05 | 0.025 |
| | | DEPT | (part by mass) | | | | | | | | | |
| | | PDE | (part by mass) | 0.2 | | 0.1 | 0.2 | | 0.1 | 0.2 | | 0.1 |
| | (G) | Filler (G-1) | (part by mass) | | 200 | 100 | | 250 | 125 | | 150 | 75 |
| | | Filler (G-2) | (part by mass) | 1.0 | 1.0 | 1.0 | 1.0 | 1.0 | 1.0 | 1.0 | 1.0 | 1.0 |
| | | BHT | (part by mass) | 0.01 | 0.05 | 0.03 | 0.01 | 0.05 | 0.03 | 0.01 | 0.05 | 0.03 |
| | | D2.6E | (part by mass) | | | | | | | | | |
| Properties | | Fluoride ion releasability | (µg/g) | 375 | | | 318 | | | 333 | | |
| | | Calcium ion releasability | (µg/g) | 180 | | | 154 | | | 186 | | |
| | | Flexural strength | (MPa) | 124 | | | 121 | | | 122 | | |
| | | Flexural modulus | (GPa) | 6.4 | | | 6.5 | | | 6.1 | | |
| | | Adhesion strength after 24 hours | (MPa) | 15 | | | 13 | | | 14 | | |
| | | Adhesion strength after TC4000 | (MPa) | 12 | | | 11 | | | 10 | | |

(continued)

**Table 1 (continued)**

| | | | | Example 4 | | | Example 5 | | | Example 6 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 50 | 50 | 50 | 50 | 80 | 65 | 50 | 80 | 65 |
| | | U4TH | (part by mass) | | 30 | 15 | | | | | | |
| | (B) | BMHPE | (part by mass) | 30 | | 15 | | | | 30 | | 15 |
| | | GDM | (part by mass) | | | | 30 | | 15 | | | |
| | (E) | POBMA | (part by mass) | 20 | 20 | 20 | 20 | 20 | 20 | | | |
| | | LMA | (part by mass) | | | | | | | 20 | 20 | 20 |
| | (C) | THP | (part by mass) | 2.0 | | 1.0 | 2.0 | | 1.0 | 2.0 | | 1.0 |
| | | BPO | (part by mass) | | | | | | | | | |
| | | CQ | (part by mass) | | 0.2 | 0.1 | | 0.2 | 0.1 | | 0.2 | 0.1 |
| | (D) | Compound (D-1) | (part by mass) | 200 | | 100 | 200 | | 100 | 200 | | 100 |
| Material | | Compound (D-2) | (part by mass) | | | | | | | | | |
| | | Compound (D-3) | (part by mass) | 5.0 | | 2.5 | 5.0 | | 2.5 | 5.0 | | 2.5 |
| | (F) | PTU | (part by mass) | | 1.5 | 0.75 | | 1.5 | 0.75 | | 1.5 | 0.75 |
| | | VOAA | (part by mass) | | 0.05 | 0.025 | | 0.05 | 0.025 | | 0.05 | 0.025 |
| | | DEPT | (part by mass) | | | | | | | | | |
| | | PDE | (part by mass) | 0.2 | | 0.1 | 0.2 | | 0.1 | 0.2 | | 0.1 |
| | (G) | Filler (G-1) | (part by mass) | | 200 | 100 | | 200 | 100 | | 200 | 100 |
| | | Filler (G-2) | (part by mass) | 1.0 | 1.0 | 1.0 | 1.0 | 1.0 | 1.0 | 1.0 | 1.0 | 1.0 |
| | | BHT | (part by mass) | 0.01 | 0.05 | 0.03 | 0.01 | 0.05 | 0.03 | 0.01 | 0.05 | 0.03 |
| | | D2.6E | (part by mass) | | | | | | | | | |
| Properties | | Fluoride ion releasability | (µg/g) | 305 | | | 367 | | | 352 | | |
| | | Calcium ion releasability | (µg/g) | 151 | | | 174 | | | 166 | | |
| | | Flexural strength | (MPa) | 126 | | | 123 | | | 112 | | |
| | | Flexural modulus | (GPa) | 6.6 | | | 6.3 | | | 5.4 | | |
| | | Adhesion strength after 24 hours | (MPa) | 13 | | | 14 | | | 12 | | |
| | | Adhesion strength after TC4000 | (MPa) | 12 | | | 12 | | | 10 | | |

**Table 2**

| | | | | Example 7 | | | Example 8 | | | Example 9 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 50 | 80 | 65 | 50 | 80 | 65 | 50 | 80 | 65 |
| | | U4TH | (part by mass) | | | | | | | | | |
| | (B) | BMHPE | (part by mass) | 30 | | 15 | 30 | | 15 | 30 | | 15 |
| | | GDM | (part by mass) | | | | | | | | | |
| | (E) | POBMA | (part by mass) | 20 | 20 | 20 | 20 | 20 | 20 | 20 | 20 | 20 |
| | | LMA | (part by mass) | | | | | | | | | |
| | (C) | THP | (part by mass) | | | | 2.0 | | 1.0 | 2.0 | | 1.0 |
| | | BPO | (part by mass) | 1.5 | | 0.75 | | | | | | |
| | | CQ | (part by mass) | | 0.2 | 0.1 | | 0.4 | 0.2 | | 0.2 | 0.1 |
| | (D) | Compound (D-1) | (part by mass) | 200 | | 100 | 80 | | 40 | | 200 | 100 |
| Material | | Compound (D-2) | (part by mass) | | | | 20 | | 10 | | | |
| | | Compound (D-3) | (part by mass) | 5.0 | | 2.5 | 5.0 | | 2.5 | 5.0 | | 2.5 |
| | (F) | PTU | (part by mass) | | | | | 1.5 | 0.75 | | 1.5 | 0.75 |
| | | VOAA | (part by mass) | | | | | 0.05 | 0.025 | | 0.05 | 0.025 |
| | | DEPT | (part by mass) | | 1.0 | 0.5 | | | | | | |
| | | PDE | (part by mass) | 0.2 | | 0.1 | 0.5 | | 0.25 | 0.2 | | 0.1 |
| | (G) | Filler (G-1) | (part by mass) | | 200 | 100 | 100 | 200 | 150 | 200 | | 100 |
| | | Filler (G-2) | (part by mass) | 1.0 | 1.0 | 1.0 | 0.5 | 1.0 | 0.75 | 1.0 | 1.0 | 1.0 |
| | | BHT | (part by mass) | 0.8 | 0.04 | 0.42 | 0.01 | 0.05 | 0.03 | 0.01 | 0.05 | 0.03 |
| | | D2.6E | (part by mass) | | | | | | | | | |
| Properties | | Fluoride ion releasability | (µg/g) | 349 | | | 312 | | | 289 | | |
| | | Calcium ion releasability | (µg/g) | 153 | | | 159 | | | 132 | | |
| | | Flexural strength | (MPa) | 108 | | | 102 | | | 112 | | |
| | | Flexural modulus | (GPa) | 5.3 | | | 5.2 | | | 5.7 | | |
| | | Adhesion strength after 24 hours | (MPa) | 11 | | | 12 | | | 12 | | |
| | | Adhesion strength after TC4000 | (MPa) | 9.3 | | | 10 | | | 9.5 | | |

(continued)

**Table 2 (continued)**

| | | | | Example 10 | | | Example 11 | | | Example 12 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 80 | 50 | 65 | 80 | 30 | 55 | 20 | 40 | 30 |
| | | U4TH | (part by mass) | | | | | | | | | |
| | (B) | BMHPE | (part by mass) | | 30 | 15 | | 70 | 35 | 30 | | 15 |
| | | GDM | (part by mass) | | | | | | | | | |
| | (E) | POBMA | (part by mass) | 20 | 20 | 20 | 20 | 0 | 10 | 20 | 20 | 20 |
| | | LMA | (part by mass) | | | | | | | | | |
| | (C) | THP | (part by mass) | 2.0 | | 1.0 | 2.0 | | 1.0 | 2.0 | | 1.0 |
| | | BPO | (part by mass) | | | | | | | | | |
| | | CQ | (part by mass) | | 0.2 | 0.1 | | 0.2 | 0.1 | | 0.2 | 0.1 |
| | (D) | Compound (D-1) | (part by mass) | 200 | | 100 | 200 | | 100 | 200 | | 100 |
| Material | | Compound (D-2) | (part by mass) | | | | | | | | | |
| | | Compound (D-3) | (part by mass) | 5.0 | | 2.5 | 5.0 | | 2.5 | 5.0 | | 2.5 |
| | (F) | PTU | (part by mass) | | 1.5 | 0.75 | | 1.5 | 0.75 | | 1.5 | 0.75 |
| | | VOAA | (part by mass) | | 0.05 | 0.025 | | 0.05 | 0.025 | | 0.05 | 0.025 |
| | | DEPT | (part by mass) | | | | | | | | | |
| | | PDE | (part by mass) | 0.2 | | 0.1 | 0.2 | | 0.1 | 0.2 | | 0.1 |
| | (G) | Filler (G-1) | (part by mass) | | 200 | 100 | | 200 | 100 | | 200 | 100 |
| | | Filler (G-2) | (part by mass) | 1.0 | 1.0 | 1.0 | 1.0 | 1.0 | 1.0 | 1.0 | 1.0 | 1.0 |
| | | BHT | (part by mass) | 0.01 | 0.05 | 0.03 | 0.01 | 0.05 | 0.03 | 0.01 | 0.05 | 0.03 |
| | | D2.6E | (part by mass) | | | | | | | 30 | 40 | 35 |
| Properties | | Fluoride ion releasability | (µg/g) | 278 | | | 283 | | | 216 | | |
| | | Calcium ion releasability | (µg/g) | 123 | | | 129 | | | 118 | | |
| | | Flexural strength | (MPa) | 122 | | | 128 | | | 128 | | |
| | | Flexural modulus | (GPa) | 6.2 | | | 6.4 | | | 6.6 | | |
| | | Adhesion strength after 24 hours | (MPa) | 13 | | | 16 | | | 11 | | |
| | | Adhesion strength after TC4000 | (MPa) | 11 | | | 13 | | | 9.2 | | |

**Table 3**

| | | | | Com parative Exam ple 1 | | | Com parative Exam ple 2 | | | Com parative Exam ple 3 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | | | | 50 | 80 | 65 | 50 | 80 | 65 |
| | | U4TH | (part by mass) | | | | | | | | | |
| | (B) | BMHPE | (part by mass) | 30 | | 15 | | | | 30 | | 15 |
| | | GDM | (part by mass) | | | | | | | | | |
| | (E) | POBMA | (part by mass) | 20 | 20 | 20 | 20 | 20 | 20 | 20 | 20 | 20 |
| | | LMA | (part by mass) | | | | | | | | | |
| | (C) | THP | (part by mass) | 2.0 | | 1.0 | 2.0 | | 1.0 | 2.0 | | 1.0 |
| | | BPO | (part by mass) | | | | | | | | | |
| | | CQ | (part by mass) | | 0.2 | 0.1 | | 0.2 | 0.1 | | 0.2 | 0.1 |
| | (D) | Compound (D-1) | (part by mass) | 200 | | 100 | 200 | | 100 | | | |
| Material | | Compound (D-2) | (part by mass) | | | | | | | | | |
| | | Compound (D-3) | (part by mass) | 5.0 | | 2.5 | 5.0 | | 2.5 | | | |
| | (F) | PTU | (part by mass) | | 1.5 | 0.75 | | 1.5 | 0.75 | | 1.5 | 0.75 |
| | | VOAA | (part by mass) | | 0.05 | 0.025 | | 0.05 | 0.025 | | 0.05 | 0.025 |
| | | DEPT | (part by mass) | | | | | | | | | |
| | | PDE | (part by mass) | 0.2 | | 0.1 | 0.2 | | 0.1 | 0.2 | | 0.1 |
| | (G) | Filler (G-1) | (part by mass) | | 200 | 100 | | 200 | 100 | 200 | 200 | 200 |
| | | Filler (G-2) | (part by mass) | 1.0 | 1.0 | 1.0 | 1.0 | 1.0 | 1.0 | 1.0 | 1.0 | 1.0 |
| | | BHT | (part by mass) | 0.01 | 0.05 | 0.03 | 0.01 | 0.05 | 0.03 | 0.01 | 0.05 | 0.03 |
| | | D2.6E | (part by mass) | 50 | 80 | 65 | | | | | | |
| | | HEMA | (part by mass) | | | | 30 | | 15 | | | |
| Properties | | Fluoride ion releasability | (µg/g) | 13 | | | 382 | | | 0 | | |
| | | Calcium ion releasability | (µg/g) | 7 | | | 192 | | | 0 | | |
| | | Flexural strength | (MPa) | 128 | | | 87 | | | 123 | | |
| | | Flexural modulus | (GPa) | 6.5 | | | 3.7 | | | 6.3 | | |
| | | Adhesion strength after 24 hours | (MPa) | 14 | | | 9.7 | | | 15 | | |
| | | Adhesion strength after TC4000 | (MPa) | 11 | | | 1.3 | | | 13 | | |

It is understood from the results in Tables 1 and 2 that the two-component type dental curable compositions of Examples containing the component (A), the component (B), the component (C), and the component (D) are excellent in ion releasability, flexural strength, flexural modulus, and adhesion durability.

On the other hand, it is understood from the results in Table 3 that the two-component type dental curable composition of Comparative Example 1 containing no component (A) has a low ion releasability. It is also understood therefrom that the two-component type dental curable composition of Comparative Example 2 containing no component (B) has a low flexural strength, a low flexural modulus, and a low adhesion durability. It is also understood therefrom that the two-component type dental curable composition of Comparative Example 3 containing no component (D) cannot achieve the ion releasability.

### Industrial Applicability

The dental curable composition can be favorably applied to a dental composite resin, and particularly to a dental filling and restorative material, a dental cement, and a dental abutment building material.

## Claims

1. A dental curable composition comprising a (meth)acrylic based monomer (A) containing two or more (meth)acryloyloxy groups and one or more urethane bond in one molecule, a (meth)acrylate ester compound (B) containing two or more (meth)acryloyloxy groups and one or more hydroxy group in one molecule, a polymerization initiator (C), and an ion releasing compound (D).

2. The dental curable composition according to claim 1, wherein the dental curable composition further comprises a mono(meth)acrylate ester compound (E) that does not contain any of a hydroxy group, an acidic group, an amino group, and an amide group in one molecule.

3. The dental curable composition according to claim 1 or 2, wherein the dental curable composition further comprises a polymerization accelerator (F).

4. The dental curable composition according to any one of claims 1 to 3, wherein the dental curable composition further comprises a filler (G).

5. The dental curable composition according to any one of claims 1 to 4, wherein the dental curable composition contains substantially no polycarboxylic acid.

6. The dental curable composition according to any one of claims 1 to 5, wherein the component (D) contains one kind selected from the group consisting of a fluoride ion releasing compound and a calcium ion releasing compound.

7. The dental curable composition according to any one of claims 1 to 6, wherein the component (C) contains a chemical polymerization initiator (C-1).

8. The dental curable composition according to claim 7, wherein the dental curable composition is separately packaged in a first material containing the component (C-1) and a second component containing a polymerization accelerator (F).

9. The dental curable composition according to claim 7 or 8, wherein the first material contains the component (D).

10. A dental filling and restorative material comprising the dental curable composition according to any one of claims 1 to 9.

11. A dental cement comprising the dental curable composition according to any one of claims 1 to 9.

12. A dental abutment building material comprising the dental curable composition according to any one of claims 1 to 9.
